(19) Europäisches Patentamt
European Patent Office
Office européen des brevets

(11) **EP 2 461 845 B1**

(12) **EUROPÄISCHE PATENTSCHRIFT**

(45) Veröffentlichungstag und Bekanntmachung des Hinweises auf die Patenterteilung:
**10.07.2013  Patentblatt 2013/28**

(21) Anmeldenummer: **10771642.5**

(22) Anmeldetag: **06.09.2010**

(51) Int Cl.:
***A61M 1/16*** *(2006.01)*

(86) Internationale Anmeldenummer:
**PCT/EP2010/005455**

(87) Internationale Veröffentlichungsnummer:
**WO 2011/026647 (10.03.2011 Gazette 2011/10)**

(54) **VORRICHTUNG ZUR EXTRAKORPORALEN BLUTBEHANDLUNG**

DEVICE FOR EXTRACORPOREAL BLOOD TREATMENT

DISPOSITIF POUR LE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **04.09.2009   DE 102009040104**

(43) Veröffentlichungstag der Anmeldung:
**13.06.2012   Patentblatt 2012/24**

(73) Patentinhaber: **B. Braun Avitum AG**
**34212 Melsungen (DE)**

(72) Erfinder:
• **AHRENS, Jörn**
**34225 Baunatal (DE)**

• **MOLL, Stefan**
**34212 Melsungen (DE)**
• **CASTELLARNAU, Alex**
**34212 Melsungen (DE)**

(74) Vertreter: **Arth, Hans-Lothar**
**ABK Patent & Trademark Attorneys**
**Jasminweg 9**
**14052 Berlin (DE)**

(56) Entgegenhaltungen:
**EP-A1- 0 706 044     EP-A1- 2 005 982**
**WO-A1-94/08641     WO-A1-99/62574**
**US-A- 5 685 988**

EP 2 461 845 B1

**Beschreibung**

Die Erfindung betrifft eine Vorrichtung zur extrakorporalen Blutbehandlung nach dem Oberbegriff des Patentanspruchs 1.

Bei Patienten mit reduzierter bzw. überhaupt keiner Nierenfunktion werden Abfallprodukte, einschließlich toxischer Substanzen, mittels einer Nierenersatzbehandlung beseitigt, wobei das Blut des Patienten über einer Blutzuführleitung vom Patienten der künstlichen Niere bzw. dem Dialysator zugeführt wird. In der künstlichen Niere bzw. dem Dialysator wird das Blut des Patienten über eine semipermeable Membran mit Dialysierflüssigkeit in Kontakt gebracht. Die Dialysierflüssigkeit enthält unterschiedliche Salze in einer solchen Konzentration, dass die Abfallprodukte, einschließlich der toxischen Substanzen, mittels Diffusion und Konvektion durch die Membran aus dem Blut des Patienten zur Dialysierflüssigkeit geführt werden. Das so von den Abfallprodukten bereinigte Blut wird über eine an dem Dialysator angeschlossenen Blutabführleitung wieder in den Blutkreislauf des Patienten zurückgeführt.

Um das Ergebnis einer Nierenersatzbehandlung quantifizieren zu können, ist es notwendig, die Effizienz der Nierenersatzbehandlung unmittelbar bzw. online zu steuern. Deshalb wurde das so genannte Kt/V-Modell entwickelt. Der Kt/V-Wert ist dabei ein Parameter zur Bestimmung der Effektivität einer Nierenersatzbehandlung, wobei die Clearance K für den Volumenstrom der gereinigten harnpflichtigen Substanzen, t für die Behandlungszeit und V für das Verteilungsvolumen des Patienten steht. Dabei sind sowohl K als auch V jeweils auf das auf das jeweilige Abfallprodukt bezogen. In der Regel wird die Effizienz bei einer Nierenersatzbehandlung anhand des Harnstoffes als Abfallprodukt beschrieben, so dass K die Harnstoffclearance und V das Harnstoffverteilungsvolumen des Patienten, welches im Wesentlichen dem Körperwassers des Patienten entspricht, beschreibt.

Aus der EP 1 083 948 A1 ist es bekannt, mit Hilfe einer im Ablauf angeordneten Messeinrichtung spektralphotometrisch unter Verwendung von UV-Strahlung und deren Absorbanz durch harnpflichtige Substanzen in der Dialysierflüssigkeit den Kt/V-Wert bzw. die Reduktionsrate RR für ein bestimmtes Abfallprodukt während der Nierenersatzbehandlung zu bestimmen. Um diesen Kt/V-Wert bzw. die Reduktionsrate bestimmen zu können, ist es notwendig, die Konzentration bzw. die dazu proportionale Absorbanz harnpflichtige Substanzen im Blut des zu therapierenden Patienten zu jedem Zeitpunkt der Nierenersatzbehandlung zu kennen. Zur Bestimmung dieser Absorbanz, wird in der EP 2 005 982 A1 vorgeschlagen, die Dialysierflüssigkeit während der Nierenersatzbehandlung so lange gegen den extrakorporalen Blutkreislauf des Patienten rezirkulieren zu lassen, bis sich Konzentrationsgleichgewicht und damit ein konstanter Wert für die Absorbanz durch harnpflichtige Substanzen in der Dialysierflüssigkeit eingestellt hat. Ist dieser konstante Wert erreicht , entspricht die Konzentration der harnpflichtigen Substanzen in der Dialysierflüssigkeit der Konzentration der harnpflichtigen Substanzen im Blut des zu behandelten Patienten, so dass auch die ermittelte Absorbanz in der Dialysierflüssigkeit gleich der im Blut des Patienten gleichsetzen kann. Durch diese Massnahme wir anstelle des Kt/V-Wertes der so genannte equilibrierte Kt/V-Wert eKt/V bestimmt, so dass der Konzentrationsausgleich der harnstoffpflichtigen Substanzen innerhalb der verschiedenen Kompartements des Patientenkörpers berücksichtigt ist.

Als problematisch hat sich bei diesem Verfahren allerdings herausgestellt, dass durch die Durchführung der Rezirkulation der Dialysierflüssigkeit gegen den extrakorparalen Blutkreislauf des Patienten auf Grund des geringer werdenden Konzentrationsgradienten während des Vorgangs, der dem Verlauf einer Aufladefunktion folgt, die Therapiedauer nicht nur unwesentlich erhöht wird, da die Einstellung des Konzentrationsgleichgewichts fünf Minuten und auch länger dauern kann. Wird dieses Verfahren mehrmals während einer Behandlung eines Patienten durchgeführt, erhöht sich die Behandlungsdauer entsprechen, was nicht nur unwirtschaftlich ist, sondern auch den Patienten zusätzlich belastet. Weiterhin problematisch ist zudem, dass bei diesem Verfahren die volle im Blut vorhandene Menge an harnpflichtigen Substanzen und damit eine entsprechend hohe Absorbanz gemessen werden muss, was den in der Therapie auftretenden Messbereich - da keine Verdünnung erfolgt - um den Faktor 3 übersteigt. Insoweit ist auch ein zusätzlicher apparativer Aufwand auf Seiten der verwendeten Messeinrichtung für die Absorbanz notwendig, um die auftretenden Absobanzen während der eigentlichen Therapie und während der Rezirkulation der Dialysierflüssigkeit bestimmen zu können.

Aufgabe der vorliegenden Erfindung ist es daher, eine Vorrichtung bereitzustellen, mit der es möglich ist, entsprechende Konzentrations- bzw. Absorbanzwerte zu bestimmen, ohne die Therapie- bzw. Behandlungszeit des Patienten derart zu verlängern, so das nicht nur ökonomischer therapiert werden kann, sonder auch eine zusätzliche Belastung des Patienten durch eine länger andauernde Therapie bzw. Behandlung minimiert wird. dabei soll auch hinsichtlich der zu Messeinrichtung für die Absorbanz der apparative Aufwand minimal gehalten werden.

Gelöst wird diese Aufgabe durch eine Vorrichtung mit den Merkmalen des Anspruchs 1. Vorteilhafte Ausgestaltungen der Erfindungen sind Gegenstand der Unteransprüche.

Ausgangspunkt der vorliegenden Erfindung ist folgender von Kaufmann et al. ("Solute disequilibrium and multicompartment modeling ", Advances in renal replacement therapy 1995;2(4):319-29, Kaufman A M; Schneditz D; Smye S; Polaschegg H D; Levin N W) gefundener Zusammenhang zwischen der Dialysatorclearance $Q_{Clearance}$ und dem Blutfluss $Q_{Blut}$ währende einer Nierenersatzbehandlung:

$$Q_{Clearance} = Q_{Blut} \cdot \left( 1 - e^{\left( -\frac{P \cdot S}{Q_{Blut}} \right)} \right) \qquad (1)$$

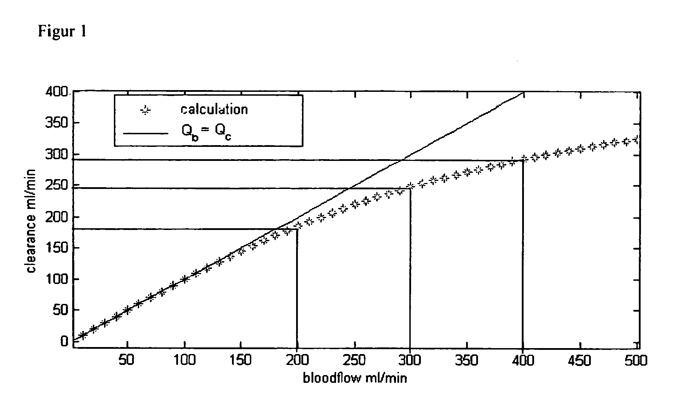

Dabei bezeichnet P die Permeabilität und S die Oberfläche des Membranmaterials eines für eine Nierenersatzbehandlung zu verwendeten Dialysators. Das Produkt P*S ist dabei eine Materialeigenschaft, welche für jeden Dialysator charakteristisch ist und in der Regel in den Datenblättern der Hersteller aufgeführt ist bzw. über die dort angegebenen Daten ermittelbar ist. In dem vorliegend in Figur 1 dargestellten Beispiel wurde mit einem P*S-Wert von 525 ml/min gerechnet, der dem einem handelsüblichen Dialysator entspricht.

Wie aus Gleichung (1) ersichtlich, wird durch einen großen Wert für P*S und einen kleinen Wert für den Blutfluss $Q_{Blut}$ erreicht, dass bei niedrigen Blutflüssen die Funktion zwischen Clearance und Blutfluss linear ist, da der Anteil der e-Funktion gegen null geht. Gleichung (1) zeigt somit, dass bei Blutflüssen < 100 ml/min die Abweichung zwischen Blutfluss und Clearance so gering ist, dass sie vernachlässigbar ist und somit der Blutfluss $Q_{Blut}$ gleich der Clearance $Q_{Clearance}$ ist.

Mit der erfindungsgemäßen Vorrichtung ist nun - wie nachfolgend dargelegt - möglich mit Hilfe der während eine Nierenersatztherapie transportierten Massen der toxischen Substanzen eine Absorbanz $A_{Blut}$ zu bestimmen:

Während der Messung wird somit die Absorbanz $A_{Dialysat}$ auf der Dialysierflüssigkeitsseite hinter dem Dialysator gemessen. Da der Massentransport des Blut durch den Dialysator gleich dem im Dialysierflüssikeit sein muss, ergibt sich folgender in den Gleichungen (2a) und (2b) beschriebene mathematischer Zusammenhang

$$A_{Blut} \cdot Q_{Clearance} = A_{Dialysat} \cdot Q_{Dialysat} \qquad (2a)$$

$$A_{Blut} = \left( \frac{A_{Dialysat} \cdot Q_{Dialysat}}{Q_{Clearance}} \right) \qquad (2b)$$

Setzt man nun in Gleichung (2b) den Wert der der Dialysatorclearance aus Gleichung (1) ein erhält man Gleichung (2c):

$$A_{Blut} = \left( \frac{A_{Dialysat} \cdot Q_{Dialysat}}{Q_{Blut} \cdot \left( 1 - e^{\left( -\frac{P \cdot S}{Q_{Blut}} \right)} \right)} \right) \qquad (2c)$$

Bei bekanntem Wert für das Produkt P*S lässt sich nun zu jedem Zeitpunkt der Therapie durch Messen der Absorbanz $A_{Dialysat}$ im Dialysierflüssigkeitsabfluss, des Dialysatflusses $Q_{Dialysat}$ und des Blutflusses $Q_{Blut}$ die Absorbanz $A_{Blut}$ bestimmen. Mit Hilfe dieses $A_{Blut}$-Wertes ist es nun möglich zu jedem Zeitpunkt der Therapie den equilibrierten Kt/V-Wert eKt/V direkt ohne wesentlich Zeitverzögerung zu bestimmen, so dass die Therapiezeit mit Hilfe der erfindungsgemäßen Vorrichtung minimiert ist.

Um nicht immer auf das Produkt P*S des verwendeten Dialysator Werte angewiesen zu sein, hat es sich als vorteilhaft erwiesen, den Wert für die e-Funktion in den Gleichungen (1) und (2c) gleich null zu setzen. Dies ist insbesondere sinnvoll, da dadurch die Rechnung der Absorbanz $A_{Blut}$ unabhängig von den verwendeten Dialysatoren ist. Dabei beträgt der Fehler bei einem Blutfluss von 100 ml/ min und einem P*S-Wert von 500 bereits weniger als 1 %, so dass damit problemlos gearbeitet werden kann.

Gleichung (1) ergibt sich damit zu:

$$Q_{Clearance} = Q_{Blut} \cdot \qquad\qquad (1b)$$

und Gleichung (2c) zu:

$$A_{Blut} = \left( \frac{A_{Dialysat} \cdot Q_{Dialysat}}{Q_{Blut}} \right) \qquad\qquad (2d)$$

und mit der Gleichung (2c oder 2d) der absolute Absorbanzwert auf der Blutseite bestimmt. Die Ultrafiltrationsrate muss dabei auf das Minimum reduziert werden, um deren Einfluss auf die Absorbanzmessung um minimieren.

Um die Einzelmessung mit anderen Messungen, insbesondere während anderer Therapiebehandlungen vergleichen zu können bzw. um einen Vergleich bei variablen Dialysierflüssigkeitsflüssen oder Ultrafiltrationsflüssen während derselben Therapiebehandlung zu ermöglichen., hat es sich als hilfreich und vorteilhaft erwiesen, die so ermittelte Absorbanz im Dialysat auf einen Standartfluss von bspw. 500ml/min zu normieren. Der formale Zusammenhang für die normierte Absobanz $A_{Normiert}$ ergibt sich dabei zu:

$$A_{Normiert} = A_{Dialysat} \cdot \left( \frac{Q_{Dialysierflüssigkeit} + Q_{UF}}{Q_{Standart}} \right) \qquad \left| Q_{Standart} = 500ml/\min \right. \quad (3)$$

Mit Hilfe der Berechnung von $A_{Blut}$ durch die Formeln 2c oder 2d von $A_{Blut}$ ist es möglich die Absorbanz bzw. die dazu proportionale Konzentration von harnpflichtigen Substanzen auf der Blutseite zur bestimmen, ohne direkt im Blutkreislauf oder durch Blutprobenentnahme Messungen durchzuführen.

Damit ist es möglich, bei geringen Blutflüssen die Annahme $Q_{Blut} = Q_{Clearance}$ zu treffen , wodurch mit der folgenden Gleichung (2c) die Absorbanz auf der Blutseite berechnet werden kann. Parallel kann natürlich bei der Kenntnis von "P·S" die Gleichung (1) direkt in der Gleichung (2d) verwendet werden.

Mit Hilfe der Absorbanz $A_{Blut}$ auf der Blutseite kann dann unter Verwendung des Wasservolumens des Patienten und der Integration der Absorbanz über den Dialysatfluss die Berechnung des eKt/V durchgeführt werden. Sofern der mittelmolekulare Anteil im Blut keinen Einfluss auf das Spektrum der Absorbanzmessung hat, kann der eKt/V ebenfalls mit Hilfe der folgenden Gleichung (4e) bestimmt werden. Sofern

Flussänderungen, beispielsweise durch die UF-Raten, erfolgen, kann dies durch die Normierung kompensiert werden.

$$Stoffmenge_{Blut\_Startpunkt} = \left(A_{Blut\_Startpunkt} \cdot V_{Startpunkt}\right) \quad (4a)$$

$$Stoffmenge_{Dialysat} = \int\left(A_{Dialysat}(t)\cdot Q_{Dialysat}(t)\right)dt \quad (4b)$$

$$A_{Blut\_Therapieende} = \left(\frac{Stoffmenge_{Blut\_Startpunkt} - Stoffmenge_{Dialysat}}{V_{Startpunkt} - V_{UF}}\right) \quad (4c)$$

$$Stoffmenge_{Blut\_Therapieende} = Stoffmenge_{Blut\_Startpunkt} - Stoffmenge_{Dialysat} \quad (4d)$$

$$eKt/V = -\ln\left(\frac{A_{Blut\_Therapieende}}{A_{Blut\_Startpunkt}}\right) \quad (4e)$$

Parallel dazu ist es möglich, den absoluten Anteil der mittelmolekularen Stoffe im Blut zu bestimmen indem während der Absorbanzmessung der konvektive Anteil mittels UF-Pumpe hinzugeschaltet wird. Dazu wird zunächst eine Messung mit reinem diffusiven Anteil durchgeführt mit Hilfe einer normierten der Absorbanz in der Dialysierflüssigkeit (5a) durch einsetzen in die Gleichung (5d) die Absorbanz von kleinmolekularen Stoffen bestimmt.

Anschließend wird durch hinzuschalten der UF-Pumpe eine erneute Messung durchgeführt und es erfolgt ebenfalls eine Normierung (5b) der Absorbanz auf den Fluss, da dieser durch Addition des Ultrafiltrationsflusses ansteigt.

Der mittelmolekulare Anteil kann mit Hilfe von Gleichung (5c) aus den beiden Messungen bestimmt werden. Grundlage dafür ist, dass die Messung des diffusiven Anteils mit einem geringen Blutfluss durchgeführt wurde, damit die Menge an kleinmolekularen Stoffen vollständig vom Blut in die Dialysierflüssigkeit übergeht ($Q_{Blut} = Q_{Clearance}$), da ansonsten beim hinzuschalten des konvektiven Transports noch vorhandene diffusiv transportierte Stoffe eine Verfälschung verursachen können, sofern man diesen nicht durch eine mathematische Korrektur kompensieren kann.

Die Bestimmung der Absorbanz der mittelmolekularen Subtanzen auf der Blutseite erfolgt durch die Gleichung (6a). Da durch die UF-Rate nur ein Teil der Mittelmolekühle des Blutflusses $Q_{Blut}$ im Gegensatz zum diffusiven Transport entzogen wird, muss der Absorbanzwert (6a) auf den gesamten Blutfluss normiert werden.

$$A_{Nomiert} = \left(A_{Dialysat} \cdot \frac{Q_{Dialysat}}{Q_{Standart}}\right) \quad | \quad Q_{Standart} = 500ml/\min \quad (5a)$$

$$A_{Nomiert+UF} = \left( A_{Dialysat+UF} \cdot \frac{Q_{Dialysat} + Q_{UF}}{Q_{S\tan dart}} \right) \quad | \quad Q_{S\tan dart} = 500 ml / \min \quad (5b)$$

$$A_{Dialysat\_MM} = \left( A_{Normiert+UF} - A_{Normiert} \right) \cdot \quad (5c)$$

$$A_{Blut} = \left( \frac{A_{Normiert} \cdot Q_{Dialysat}}{Q_{Blut}} \right) \quad | \quad Q_{Clearance} = Q_{Blut} \quad (5d)$$

$$A_{Blut\_MM} = \left( A_{Normiert+UF} - A_{Normiert} \right) \cdot \left( \frac{Q_{Blut}}{Q_{UF}} \right) \quad (6a)$$

Parallel dazu kann der eKt/V (6f) ebenfalls für den mittelmolekularen Teil berechnet werden, indem durch aktivieren und deaktivieren der UF-Pumpe der entfernte mittelmolekulare Anteil (6c) bestimmt wird und mit dem zu Beginn berechneten absoluten mittelmolekularen Anteil (6b) im Blut des Patienten verrechnet wird (Gleichung 6a-6f):

$$Stoffmenge_{Blut\_MM\_Startpunkt} = \left( A_{Blut\_MM\_Startpunkt} \cdot V_{Startpunkt} \right) \quad (6b)$$

$$Stoffmenge_{Dialysat\_MM} = \int \left( A_{Dialysat\_MM}(t) \cdot Q_{S\tan dart} \right) dt \quad (6c)$$

$$A_{Blut\_MM\_Therapieende} = \left( \frac{Stoffmenge_{Blut\_MM\_Startpunkt} - Stoffmenge_{Dialysat\_MM}}{V_{Startpunkt} - V_{UF}} \right) \quad (6d)$$

$$Stoffmenge_{Blut\_MM\_Therapieende} = Stoffmenge_{Blut\_MM\_Startpunkt} - Stoffmenge_{Dialysat\_MM} \quad (6e)$$

$$eKt / V_{konvektion} = -\ln \left( \frac{A_{Blut\_MM\_Therapieende}}{A_{Blut\_MM\_Startpunkt}} \right) \quad (6f)$$

Zur Ermittelung des diffusiven Anteils über das Integral über die Therapie muss daher während der Therapie eine Messphase mit reinem diffusiven Anteil ohne Ultrafiltration eingefügt werden.

Zur Ermittlung des reinen diffusiven Anteils wird die UF-Rate auf das Minimum reduziert, der Blutfluss angepasst und der Absorbanzwert zu diesem Zeitpunkt aufgenommen. Zur Ermittlung des konvektiven Anteils wird im Anschluss der konvektive Anteil wieder hinzugeschaltet, um die Absorbanz des konvektiven Anteils aus deren Differenz (5c) zu berechnen.

Damit ist es möglich aus dem jeweils gefitteten Verlauf der diffusiven Absorbanz (5a) über die Therapie, dem konvektiven Verlauf der Absorbanz (5c) und dem eigentlichen Therapieverlauf der Absorbanz aus konvektiven und diffusiven Anteilen den Kt/V bzw. den eKt/V zu berechnen. Sofern die einstellte Ultrafiltration während der konvektiven Messphase der normalen UF-Rate der Therapie entspricht kann die beibehalten werden, sofern der Signalhub ausreicht. Das Verhältnis der diffusiven Phase (UF-Rate=0) und der konvektiven Phase (UF-Rate an) sollte aber idealer weise über den Zeitraum der Messung im Mittel der in der Therapie Standard eingestellten UF-Rate entsprechen um weitere Anpassungen zum erreichen des Zielvolumens zu verhindern.

Zur Bestimmung der effektiven Clearance muss zuvor die Absorbanz $A_{Blut}$ ohne Ultrafiltration bestimmt werden. Danach ist es möglich die Clearance wieder im normalen Therapiebetrieb ebenfalls ohne Ultrafiltration direkt im Anschluss zu bestimmen. Unter der Vorraussetzung, das sich im Anschluss der Wert $A_{Blut}$ noch mit der selben Konzentration im Blut befindet, kann mit der Formel (7) die effektive Clearance ausgerechnet werden.

In dieser effektiven Clearance werden unter anderem die Auswirkungen einer Verklottung und einer Rezirkulation im Shunt mit erfasst, wodurch Rückschlüsse auf die Therapieleistung geschlossen werden kann. Dem gegenüber können die Verfahren, die auf Änderungen der Leitfähigkeit basieren lediglich die Dialysatorclearance bestimmen.

$$Q_{Clearance} = \frac{A_{Dialysat\_normiert} \cdot Q_{Dialysat\_S\tan dart}}{A_{Blut}} \qquad (7)$$

Es zeigt:

Figur 1:    graphischer Zusammenhang der Clearance eines Dialysators und des Blutflusses

**Patentansprüche**

1. Vorrichtung zur extrakorporalen Blutbehandlung mit

   - einem Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabführleitung mit dem Blutkreislauf eines Patienten verbindbar ist,
   - einem Zulauf für frische Dialysierflüssigkeit,
   - einem Ablauf für verbrauchte Dialysierflüssigkeit,
   - einem Mittel zur Bestimmung eines Flusses $Q_{Blut}$ in der Blutzuführ- bzw. Blutabführleitung,
   - einem Mittel zur Bestimmung eines Flusses $Q_{Dialysat}$ im Dialysierflüssigkeitsweg
   - einer in dem Ablauf angeordneten Messeinrichtung zur Bestimmung der Absorbanz $A_{Dialysat}$ der durch den Ablauf fließenden verbrauchten Dialysierflüssigkeit, wobei die Messeinrichtung wenigstens eine Strahlungsquelle und ein Detektorsystem zur Detektion der Intensität der elektromagnetischen Strahlung aufweist,

   **dadurch gekennzeichnet, dass**

   - ein Speicher, in welchem die Formel

$$A_{Blut} = \left( \frac{A_{Dialysat} \cdot Q_{Dialysat}}{Q_{Blut}} \right)$$

für die eine Absorbanz $A_{Blut}$ abgelegt ist, und
- ein Rechenwerk, mit dem die Absorbanz $A_{Blut}$ in der Blutzuführ- bzw. Blutabführleitung unter Zuhilfenahme der Absorbanz $A_{Dialysat}$, des Flusses $Q_{Dialysat}$ und des Flusses $Q_{Blut}$ bestimmbar ist,

vorgesehen sind.

2. Vorrichtung nach Anspruch 1, **dadurch gekennzeichnet, dass** das Rechenwerk als Mikroprozessor ausgebildet ist.

3. Vorrichtung nach einem der vorherigen Ansprüche, **dadurch gekennzeichnet, dass** die Strahlungsquelle eine im Wesentlichen engbandige elektromagnetische Strahlung abstrahlt.

4. Vorrichtung nach einem der vorherigen Ansprüche, **dadurch gekennzeichnet, dass** Strahlungsquelle eine im Wesentlichen monochromatische elektromagnetische Strahlung abstrahlt.

5. Vorrichtung nach einem der vorherigen Ansprüche, **dadurch gekennzeichnet, dass** Strahlungsquelle als Leuchtdiode ausgebildet ist.

6. Vorrichtung nach einem der vorherigen Ansprüche, **dadurch gekennzeichnet, dass** die Mittel zur Bestimmung eines Flusses $Q_{Blut}$ in der Blutzuführ- bzw. Blutabführleitung und/oder die Mittel zur Bestimmung eines Flusses $Q_{Dialysat}$ im Dialysierflüssigkeitsweg zur Bestimmung von Flüssen zwischen 0 ml/min und 1000ml/min ausgebildet sind.

7. Vorrichtung nach einem der vorherigen Ansprüche, **dadurch gekennzeichnet, dass** die Mittel zur Bestimmung eines Flusses $Q_{Blut}$ in der Blutzuführ- bzw. Blutabführleitung und/oder die Mittel zur Bestimmung eines Flusses $Q_{Dialysat}$ im Dialysierflüssigkeitsweg als Pumpe mit einstellbarer Flussrate bzw. direkte Flussmesseinrichtung ausgebildet sind.

8. Vorrichtung nach einem der vorherigen Ansprüche, **dadurch gekennzeichnet, dass** das Rechenwerk dafür vorgesehen ist mit Hilfe der Gleichung

$$eKt/V = -\ln\left(\frac{A_{Blut\_Therapieende}}{A_{Blut\_Startpunkt}}\right)$$

den equillibrierten eKt/-Wert zu bestimmen.

9. Vorrichtung nach einem der vorherigen Ansprüche, **dadurch gekennzeichnet, dass** das Rechenwerk dafür vorgesehen ist mit Hilfe der Gleichung

$$A_{Blut\_MM} = \left(A_{Normiert+UF} - A_{Normiert}\right) \cdot \left(\frac{Q_{Blut}}{Q_{UF}}\right)$$

die zur Konzentration der Mittelmoleküle im Blut proportionale Absorbanz der Mittelmoleküle im Blut zu bestimmen.

10. Vorrichtung nach einem der vorherigen Ansprüche, **dadurch gekennzeichnet, dass** das Rechenwerk dafür vorgesehen ist mit Hilfe der Gleichung

$$eKt/V_{Konvektion} = -\ln\left(\frac{A_{Blut\_MM\_Therapieende}}{A_{Blut\_MM\_Startpunkt}}\right)$$

den equillibrierten eKt/-Wert für den mittelmolekularen Anteil zu bestimmen.

**Claims**

1. Apparatus for extracorporeal treatment of blood with

   - a dialyzer which is separated by a semipermeable membrane into a first and a second chamber, wherein the first chamber is disposed in a dialysis fluid pathway and the second chamber is connectable to the blood circulation of a patient by means of a blood inlet and a blood outlet,
   - a feed for fresh dialysis fluid,
   - a discharge for spent dialysis fluid,
   - a means for determining a flow $Q_{Blood}$ in the blood inlet respectively the blood outlet,
   - a means for determining a flow $Q_{Dialysate}$ in the dialysis fluid pathway,
   - a measuring device disposed in the discharge for determination of the absorption $A_{Dialysate}$ of the spent dialysis fluid flowing through the discharge, wherein the measuring device has at least one radiation source and a detector system for detection of the intensity of the electromagnetic radiation,

   **characterized by**
   being provided with

   - a memory, wherein the formula

   $$A_{Blood} = \left( \frac{A_{Dialysate} \cdot Q_{Dialysate}}{Q_{Blood}} \right)$$

   is stored for the absorption $A_{Blood}$, and
   - an arithmetic unit for determination of the absorption $A_{Blood}$ in the blood inflow conduit respectively the blood outflow conduit using the absorption $A_{Dialysate}$, the flow $Q_{Dialysate}$, and the flow $Q_{Blood}$.

2. Apparatus according to claim 1, **characterized in that** the arithmetic unit is designed as a microprocessor.

3. Apparatus according to any previous claim, **characterized in that** the radiation source emits an essentially narrow-banded electromagnetic radiation.

4. Apparatus according to any previous claim, **characterized in that** the radiation source emits an essentially monochromatic electromagnetic radiation.

5. Apparatus according to any previous claim, **characterized in that** the radiation source is designed as a light emitting diode.

6. Apparatus according to any previous claim, **characterized in that** the means for determination of a flow $Q_{Blood}$ in the blood inlet respectively the blood outlet and/or the means for determination of a flow $Q_{Dialysate}$ in the dialysis fluid pathway are designed for the determination of flows between 0 ml/min and 1000 ml/min.

7. Apparatus according to any previous claim, **characterized in that** the means for determination of a flow $Q_{Blood}$ in the blood inlet respectively the blood outlet and/or the means for determination of a flow $Q_{Dialysate}$ in the dialysis fluid pathway are designed as a pump with adjustable flow rate respectively as direct flow measuring device.

8. Apparatus according to any previous claim, **characterized in that** the arithmetic unit is provided for determination of the equilibrated eKt/V-value using the formula

$$eKt/V = -\ln \left( \frac{A_{Blood\_end\ of\ therapy}}{A_{Blood\_starting\ point}} \right)$$

9. Apparatus according to any previous claim, **characterized in that** the arithmetic unit is provided for determination of the absorption of the medium molecules in the blood, which is proportional to the concentration of the medium molecules in the blood using the formula

$$A_{\text{Blood\_MM}} = (A_{\text{Standard+UF}} - A_{\text{Standard}}) \cdot \left(\frac{Q_{\text{Blood}}}{Q_{\text{UF}}}\right).$$

10. Apparatus according to any previous claim, **characterized in that** the arithmetic unit is provided for determination of the equilibrated eKt/V - value for the medium molecular proportion using the formula

$$\text{eKt/V}_{\text{convection}} = -\ln\left(\frac{A_{\text{Blood\_MM\_end of therapy}}}{A_{\text{Blood\_MM\_starting point}}}\right).$$

**Revendications**

1. Appareil pour le traitement extracorporel du sang comprenant:

   - un dialyseur qui est séparé dans une première et une seconde chambre par une membrane semi-perméable, dans lequel la première chambre est disposée sur le trajet du liquide de dialyse et la deuxième chambre peut être connectée à la circulation du sang d'un patient au moyen d'un tuyau d'entrée du sang et d'un tuyau de sortie du sang,
   - un système d'alimentation pour le liquide frais de dialyse,
   - un system d'évacuation pour le sang usé de dialyse,
   - un moyen de détermination du flux $Q_{\text{Sang}}$ dans le tuyau d'entrée du sang et respectivement, dans le tuyau de sortie du sang,
   - un moyen de détermination du flux $Q_{\text{Dialysat}}$ dans le trajet du liquide de dialyse,
   - un dispositif de mesure disposé dans le système d'évacuation pour déterminer l'absorption $A_{\text{dialysat}}$ du sang usé de dialyse s'écoulant dans le système d'évacuation, ledit dispositif de mesure ayant au moins une source de rayonnement et un système de détection pour la détection de l'intensité du rayonnement électromagnétique,

   **caractérisé par**,
   qu'il est prévu

   - d'une mémoire, dans laquelle la formule

$$A_{Sang} = \left(\frac{A_{Dialysat} * Q_{Dialysat}}{Q_{Sang}}\right)$$

   est sauvegardée pour l'absorption $A_{\text{Sang}}$, et
   - d'une unité arithmétique pour déterminer l'absorption $A_{\text{Sang}}$ dans le tuyau d'entrée du sang et respectivement dans le tuyau de sortie du sang a l'aide de l'absorption $A_{\text{Dialysat}}$, du flux $Q_{\text{Dialysat}}$, et du flux $Q_{\text{Sang}}$.

2. Appareil selon revendication 1, **caractérisé en ce que** l'unité arithmétique est conçue comme un microprocesseur.

3. Appareil selon une des revendications antérieures, **caractérisé en ce que** la source de rayonnement émet un rayonnement électromagnétique à bande essentiellement étroite.

4. Appareil selon une des revendications antérieures, **caractérisé en ce que** la source de rayonnement émet un rayonnement électromagnétique essentiellement monochromatique.

**5.** Appareil selon une des revendications antérieures, **caractérisé en ce que** la source de rayonnement est conçue comme une diode électroluminescente.

**6.** Appareil selon une des revendications antérieures, **caractérisé en ce que** le moyen de détermination du flux $Q_{Sang}$ dans le tuyau d'entrée du sang et respectivement dans le tuyau de sortie du sang et/ou le moyen de détermination du flux $Q_{Dialysat}$ dans le trajet du liquide de dialyse sont conçus pour la détermination des flux compris entre 0 ml/min et 1000 ml/min.

**7.** Appareil selon une des revendications antérieures, **caractérisé en ce que** le moyen de détermination du flux $Q_{Sang}$ dans le tuyau d'entrée du sang et respectivement dans le tuyau de sortie du sang et/ou le moyen de détermination du flux $Q_{Dialysat}$ dans le trajet du liquide de dialyse sont conçus comme une pompe a débit réglable respectivement comme un dispositif de mesure directe du flux.

**8.** Appareil selon une des revendications antérieures, **caractérisé en ce que** l'unité arithmétique est prévue pour la détermination de la valeur équilibrée eKt/V selon la formule:

$$eKt/V = -\ln\left(\frac{A_{Sang\_fin\,de\,thérapie}}{A_{Sang\_point\,de\,depart}}\right).$$

**9.** Appareil selon une des revendications antérieures, **caractérisé en ce que** l'unité arithmétique est prévue pour la détermination de l'absorption des molécules moyennes du sang, qui est proportionnelle avec la concentration des molécules moyennes dans le sang selon la formule:

$$A_{Sang\_MM} = (A_{normalisée+UF} - A_{normalisée}) * \left(\frac{Q_{Sang}}{Q_{UF}}\right).$$

**10.** Appareil selon une des revendications précédentes, **caractérisé en ce que** l'unité arithmétique est prévue pour la détermination de la valeur équilibrée eKt/V de la proportion moléculaire moyenne selon la formule:

$$eKt/V_{convection} = -\ln\left(\frac{A_{Sang\_MM\_fin\,de\,thérapie}}{A_{Sang\_MM\_point\,de\,depart}}\right).$$

Figur 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1083948 A1

- EP 2005982 A1

**In der Beschreibung aufgeführte Nicht-Patentliteratur**

- **KAUFMANN et al.** Solute disequilibrium and multicompartment modeling. *Advances in renal replacement therapy,* 1995, vol. 2 (4), 319-29